# EUROPEAN PATENT APPLICATION

(11) **EP 2 722 019 A1**
(43) Date of publication of application: **23.04.2014**
(21) Application number: 11867881.2
(22) Date of filing: 15.06.2011
(51) Int. Cl.: A61C 3/02, A61B 17/16, A61B 17/17, A61C 8/00

(54) **DRILL BIT FOR REMOVING AN IMPLANT COUPLING SCREW AND GUIDE SOCKET THEREOF**

(71) Applicant: Neobiotech, Co., Ltd, Seoul 152-789 (KR)
(72) Inventor: HEO, Young Ku, Seoul 138-829 (KR)
(74) Representative: Arth, Hans-Lothar
(86) International application number: PCT/KR2011/004369
(87) International publication number: WO 2012/173291

(57) **Abstract**

The present invention discloses a drill bit for removing implant fastening screw which comprises the hole drilling drill bit 110 for axially drilling a hole in the fastening screw which broken or damaged in an implant and the screw lifting drill bit 130 for being firmly fixed the hole of the fastening screw and for lifting the fastening screw, and a guiding socket 120 which comprises the fastening portion 121 for being firmly fastened in the upper end portion of an implant in which a broken or damaged fastening screw is remained and a guiding portion 122 for guiding the hole drilling drill bit 110 to meet the central axis of the fastening screw and the central axis of the hole drilling drill bit 110.

## Description

### Technical Field

The present invention relates to a drill bit for removing an implant fastening screw and a guiding socket thereof, and more particularly, the present invention relates to the drill bit for removing the implant fastening screw and the guiding socket thereof enabling to easily and definitely remove a fastening screw which is broken or damaged in an implant without the damage of the implant.

### Background Art

A dental implant is an artificial tooth to substitute a natural tooth partially or wholly lost, and it is composed of a fixture fixedly implanted in an alveolar bone, an abutment fixedly connected on the fixture to be exposed out of gum, and an artificial tooth fixedly connected on the abutment with dental cement.

The abutment is generally connected on the fixture by a small and thin fastening screw, but the small and thin fastening screw is occasionally broken in case that over torque is given to the fastening screw to be tightly connected to the abutment, or in case that over torque is given to the fastening screw by the masticatory force caused by masticatory movement. If the fastening screw is broken like this, the connecting force between the fixture and the abutment is greatly decreased and the implant can be not fulfilled its own function.

In this case, the broken fastening screw should be removed and the fixture and the abutment should be connected by a new fastening screw.

When the broken fastening screw is removed, the partial upper portion thereof may be removed by a fastening tool because there is a screw head having a fastening groove but the remained portion thereof in the fixture may be not removed by a fastening tool because there is no fastening groove.

Also, even the fastening screw is not broken, when the hexagonal shaped fastening groove formed on its head is damaged because a fastening tool is not fit or over torque is given and so on, the damaged screw may not be removed using existing fastening tools even it should be removed and substituted by new one.

If the broken or damaged fastening screw may be not removed, it is raised a troublesome problem in which the whole implant should be removed and the work to implant a new implant should be done.

### Disclosure of Invention

### Technical Goals

The present invention is created to solve the above mentioned conventional problem, and one goal of the present invention is to provide the drill bit for removing an implant fastening screw enabling to easily and definitely remove the fastening screw which is broken or damaged in an implant without the damage of the implant by drilling a hole on the remained screw in the implant and by tightly fixing the drill bit in the hole in the direction in which the fastening screw is released.

Further, another goal of the present invention is to provide a guiding socket for guiding the movement of the drill bit such that the hole is drilled to meet the central axis of the fastening screw when the hole is drilling on the fastening screw.

### Technical solutions

To accomplish the aim of the present invention, the present invention provides a drill bit for removing implant fastening screw comprising a hole drilling drill bit for axially drilling a hole in the fastening screw which is broken or damaged in an implant and a screw lifting drill bit for being firmly fixed the hole of the fastening screw and for lifting the fastening screw.

The present invention is characterized in that the hole drilling drill bit is provided with a hole drilling portion to drill the hole and a cooling water supplying portion to supply the cooling water to the hole drilling portion to cool the heat produced during the drilling of the hole, which are integrally provided in order, further characterized in that an entrance restrain portion to restrain the entrance depth of the hole drilling portion to drill the hole is provide to be integral very after the cooling water supplying portion, and still further characterized in that a hand-piece connecting portion to be mounted to a working tool like a hand-piece is further provide to be integral very after the entrance restrain portion.

Herein the present invention is characterized in that the hole drilling portion is formed with a pair of cutting blades for drilling the hole on the fastening screw, the cutting blades being opposite with each other on the end surface thereof, and further characterized in that the hole drilling portion is formed with a pair of channels for discharging the drilled chips of the fastening screw, the channels being connected to the pair of cutting blades and twisted in longitudinal direction on the side surface thereof.

Also, the present invention is characterized in that the cooling water supplying portion is spirally formed with a cooling water supplying groove, and the spiral cooling water supplying groove is formed to have the opposite direction of the direction in which the pair of cutting blades or the pair of channels is formed such that the cooling water is well supplied.

The present invention is characterized in that the screw lifting drill bit is provided with a hole fastening portion to be fastened to the hole formed in the fastening screw, and the diameter of the hole fastening portion is greater than that of the hole, further characterized in that the hole fastening portion is formed with side cutting blades which cut the inner periphery surface of the hole to form texture on the inner periphery surface and side fixing blades which burrow the texture to be fixed, on the outer periphery surface thereof, still further characterized in that the portion formed with the side cutting blades and the side fixing blades has a tapered shape to be narrower towards its end, and still further characterized in that the tapered angle is preferably below 15°.

The present invention provides a drill bit for removing an implant fastening screw including a screw removing portion to remove the fastening screw broken or damaged in an implant, the screw removing portion is composed of a pair of cutting blades for axially drilling a hole on the fastening screw, the cutting blades being opposite with each other on the end surface thereof; a pair of channels for outwardly discharging the chips produced during drilling on the side surface thereof, the channels being connected to the pair of cutting blades and being twisted in longitudinal direction on the side surface thereof; and a pair of side fixing blades for burrowing in the inner periphery surface of the hole drilled by the pair of cutting blades to form texture and being firmly fixed into the hole, the side fixing blades formed to be between the pair of channels.

Herein it is characterized in that the pair of cutting blades does the cutting work in the direction that the fastening is released, and the pair of channels is twisted in the direction that the fastening is released.

The present invention provides a drill bit for removing implant fastening screw including a screw removing portion to remove the fastening screw broken or damaged in an implant, the screw removing portion is composed of a pair of cutting blades for axially drilling a hole on the fastening screw, the cutting blades being contrary to each other on the end surface thereof; a pair of channels for outwardly discharging the chips produced during drilling on the side surface thereof, the channels being connected to the pair of cutting blades and being straight in longitudinal direction on the side surface thereof; and a pair of side fixing blades for burrowing in the inner periphery surface of the hole drilled by the pair of cutting blades to form texture and being firmly fixed in the hole, the side fixing blades formed to be between the pair of channels.

Herein it is characterized in that the pair of cutting blades does the cutting work in the direction that the fastening is released.

The present invention provides a drill bit for removing an implant fastening screw including a screw removing portion to remove the fastening screw broken or damaged in an implant, the screw removing portion is composed of a pair of cutting blades for axially drilling a hole on the fastening screw, the cutting blades being contrary to each other on the end surface thereof; and a pair of channels for outwardly discharging the chips produced during drilling on the side surface thereof, the channels being connected to the pair of cutting blades and being straight in longitudinal direction on the side surface thereof, wherein the screw removing portion is formed to be tapered to have an angle of below 15°.

Herein it is characterized in that the pair of cutting blades has a edge angle of below 90° to increase the cutting force.

The present invention provides a drill bit for removing an implant fastening screw including a screw removing portion to remove the fastening screw broken or damaged in an implant, the screw removing portion is hollow and composed of a pair of cutting blades for axially drilling one annular hole on the fastening screw, the cutting blades being contrary to each other on the end surface thereof; a pair of channels for outwardly discharging the chips produced during drilling on the side surface thereof, the channels being connected to the pair of cutting blades and being straight in longitudinal direction on the side surface thereof; and a pair of side fixing blades for burrowing in the inner periphery surface of the annular hole drilled by the pair of cutting blades to form texture and being firmly fixed into the hole, the side fixing blades formed to be between the pair of channels.

Herein it is characterized in that the pair of cutting blades has a edge angle of below 90° to increase the cutting force.

The present invention provides a guiding socket comprising a fastening portion for being firmly fastened in the upper end portion of an implant in which a broken or damaged fastening screw is remained; and a guiding portion for guiding a hole drilling drill bit to fit the central axis of the fastening screw and the central axis of the hole drilling drill bit.

The present invention is characterized in that the fastening portion has a hollow cylindrical shape, and it is formed with threads on the outer periphery surface to be corresponded to the threads formed in the inner periphery surface of the implant, further characterized in that the guiding portion is formed with a hexagonal shaped installing recess to be inserted with a hexagonal shaped installing protrusion formed on the working tool, the guiding portion being on the inner periphery surface of the end portion thereof, still further characterized in that the threads has the length of 0.2mm∼5mm, and still further characterized in that the fastening portion is formed with sharp protrusions for firmly hold the upper end surface of the fastening screw, the fastening portion being on the end surface following to the circumference thereof.

### Effect of the Invention

According to the present invention, because a hole is drilled on the broken or damaged fastening screw remained in the implant and the drill bit is tightly fixed in the hole in the direction in which the fastening screw is released, the broken or damaged fastening screw may be easily and definitely removed without the damage of the implant.

Further, because the movement of the drill bit is guided such that the hole is drilled to meet the central axis of the fastening screw when the hole is drilling on fastening screw, the work to remove the remained broken or damaged fastening screw in the implant may be easily and correctly done.

### Brief Description of Drawings

FIG. 1 is a perspective view illustrating a hole drilling drill bit of the drill bit for removing an implant fastening screw according to one preferred embodiment of the present invention;
FIG. 2 is a perspective view illustrating a guiding socket of the hole drilling drill bit according to one preferred embodiment of the present invention;
FIG. 3 is a sectional view obtained following to the 'A-A' line in FIG. 2;
FIGs. 4A and 4B are sectional views illustrating a work to drill a hole on the broken or damaged fastening screw remained in the implant using the hole drilling drill bit in FIG. 1 and the guiding socket in FIG. 2;
FIG. 5 is a perspective view illustrating a screw lifting drill bit of the drill bit for removing an implant fastening screw according to one preferred embodiment of the present invention;
FIGs. 6A and 6B are sectional views illustrating a work to remove the broken or damaged fastening screw from the implant using the lifting drill bit in FIG. 5;
FIG. 7 is a perspective view partially illustrating a drill bit for removing implant fastening screw according to another preferred embodiment of the present invention;
FIGs. 8A and 8B are sectional views illustrating a work to remove the broken or damaged fastening screw remained the implant using the drill bit for removing implant fastening screw in FIG. 7 and the guiding socket in FIG. 2;
FIG. 9 is a perspective view partially illustrating a drill bit for removing an implant fastening screw according to still another preferred embodiment of the present invention;
FIGs. 10A and 10B are sectional views illustrating a work to remove the broken or damaged fastening screw remained the implant using the drill bit for removing the implant fastening screw in FIG. 9 and the guiding socket in FIG. 2;
FIG. 11 is a perspective view partially illustrating a drill bit for removing an implant fastening screw according to still another preferred embodiment of the present invention;
FIGs. 12A and 12B are sectional views illustrating a work to remove the broken or damaged fastening screw remained the implant using the drill bit for removing the implant fastening screw in FIG. 11 and the guiding socket in FIG. 2;
FIG. 13 is a perspective view partially illustrating a drill bit for removing an implant fastening screw according to still another preferred embodiment of the present invention; and
FIGs. 14A and 14B are sectional views illustrating a work to remove the broken or damaged fastening screw remained the implant using the drill bit for removing the implant fastening screw in FIG. 13 and the guiding socket in FIG. 2.

### Best Mode for Carrying Out the Invention

Reference will now be made in detail to embodiments of the present invention, examples of which are illustrated in the accompanying drawings, wherein like reference numerals refer to the like elements throughout. The embodiments are described below in order to explain the present invention by referring to the figures.

Hereinafter, the drill bit for removing an implant fastening screw and the guiding socket thereof according to the preferred embodiments of the present invention are explained in detail referring to the accompanying drawings.

The drill bit for removing an implant fastening screw according to one embodiment of the present invention is comprised of a hole drilling drill bit used to drill a hole on the broken or damaged fastening screw remained in an implant and a screw lifting drill bit used to be firmly fastened to the hole and to lift the fastening screw.

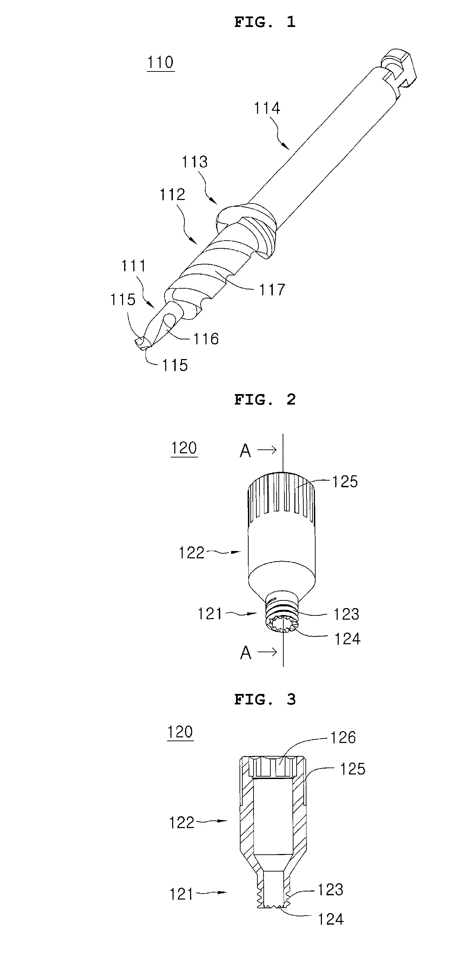

Fist, referring to FIG. 1, the hole drilling drill bit used to drill a hole on the broken or damaged fastening screw remained in an implant will be explained. FIG. 1 is a perspective view illustrating the hole drilling drill bit of the drill bit for removing the implant fastening screw according to one preferred embodiment of the present invention.

As shown in FIG. 1, the hole drilling drill bit 110 according to the present invention comprises the hole drilling portion 111, the cool water supplying portion 112, the entrance restrain portion 113 and the hand-piece connecting portion 114, which are integrally formed in order.

The hole drilling portion 111 is for drilling a hole on the broken or damaged fastening screw remained in an implant, and it has a thin and short cylindrical shape. The hole drilling portion 111 is formed with a pair of cutting blades 115 to be opposite with 180° on the end surface thereof for drilling the hole on the fastening screw, and also formed with a pair of channels 116 on the side surface thereof to be connected to the pair of cutting blades 115 and to be twisted in longitudinal direction. At this time, the pair of cutting blades 115 is formed to do the cutting work in the direction that the fastening screw is released, and the pair of channels 116 is also formed to be twisted in the same direction.

The cooling water supplying portion 112 is for supplying the cooling water to cool the heat produced during the drilling of the hole, and it has a thick and long cylindrical shape. The cooling water supplying portion 112 is spirally formed with a cooling water supplying groove 117 following to the outer periphery surface thereof to supply the cool water from outside to the hole drilling portion 111 and the surroundings of the cutting work. At this time, the spiral cooling water supplying groove 117 is formed in the direction which the fastening screw is wound, that is, in the opposite direction of the direction which the pair of cutting blades 115 or the pair of channels 116 is formed. To do like this, the cool water can be much well supplied while the hole is drilled.

The entrance restraint portion 113 is for restraining the entrance depth of the hole drilling portion 111 to drill the hole, and it is formed to have the larger diameter than the cool water supplying portion 112 and to be stepped. In this embodiment, the entrance depth of the drill bit 110 is restrained by the entrance restraint portion 113 which is stepped from the cool water supplying portion 112, but alternatively it may be marked to numerically show the entrance depth such that the user can easily know the entrance depth and then control the depth.

The hand-piece connecting portion 114 is for helping the drill bit 110 to be mounted to a working tool like a hand-piece.

Next, referring to FIGs. 2 and 3, it is explained the guiding socket to guide the hole drilling drill bit in FIG. 1 in the implant to be well installed. FIG. 2 is a perspective view illustrating a guiding socket of the hole drilling drill bit according to one preferred embodiment of the present invention, and FIG. 3 is a sectional view obtained following to the 'A-A' line in FIG. 2.

As shown in FIGs. 2 and 3, the guiding socket 120 according to the present invention includes a fastening portion 121 and the guiding portion 122, which are integrally formed in order.

The fastening portion 121 is for being firmly fastened the upper end portion of the implant in which the broken or damaged fastening screw is remained, and it has a thin and short cylindrical shape. The fastening portion 121 is formed with threads 123 on the outer periphery surface to be corresponded to the threads formed in the inner periphery surface of the implant to which the fastening screw is fastened, and also formed with sharp protrusions 124 on the end surface following to the circumference thereof. The threads 123 are formed to have the length of 0.2mm∼5mm, and the sharp protrusions 124 may be diamond particles to be attached the end surface thereof. The sharp protrusions 124 firmly hold the fastening screw such that the fastening screw is not rotated together with the drill bit 110 when the fastening screw is drilled by the hole drilling drill bit 110.

The guiding portion 122 is for guiding the hole drilling drill bit 110 to fit the central axis of the fastening screw and the central axis of the hole drilling drill bit 110, and it has a thick and long hollowed cylindrical shape. The guiding portion 122 is formed with a plurality of operation recesses 125 on the outer periphery surface of the end portion thereof to help the user to easily operate the guiding socket 120, and also formed with a hexagonal shaped installing recess 126 on the inner periphery surface of the end portion thereof. The hexagonal shaped installing recess 126 will be inserted with a hexagonal shaped installing protrusion formed on the working tool to firmly hold and to rotate the guiding socket 120 when the guiding socket 120 is fixed to the implant.

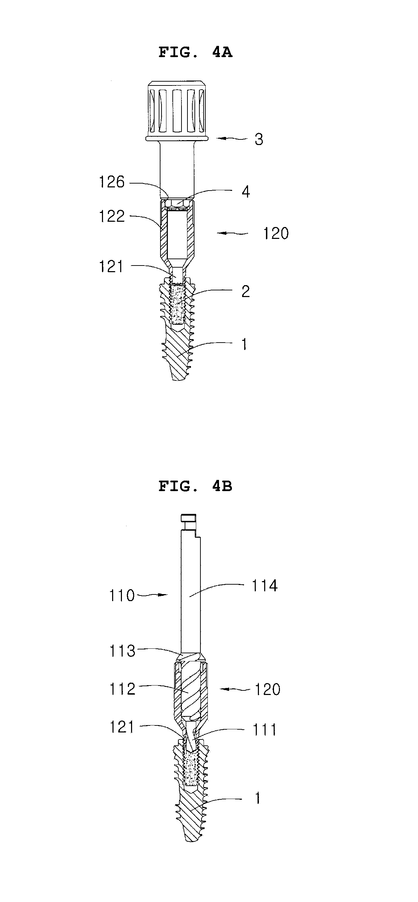

Next, referring to FIGs. 4A and 4B, it is explained the work to drill a hole on the broken or damaged fastening screw remained in the implant using the hole drilling drill bit in FIG. 1 and the guiding socket in FIG. 2. FIGs. 4A and 4B are sectional views illustrating a work to drill a hole on the broken or damaged fastening screw remained in the implant using the hole drilling drill bit in FIG. 1 and the guiding socket in FIG. 2.

As shown FIG. 4A, the guiding socket 120 is firmly fastened on the upper end portion of the implant 1 in which the broken fastened screw 2 is remained. At this time, the user holds the portion formed with the plurality of operation recesses 125 with his fingers, pushes the fastening portion 121 into the upper end portion of the implant 1, and then rotates the guiding socket 120 such that the threads formed on the outer periphery surface of the fastening portion 121 is screwed with the threads formed on the inner periphery surface of the implant 1. After that, the hexagonal shaped installing protrusion formed on the working tool 3 is inserted in the hexagonal shaped installing recess 126 formed on the guiding portion 122 of the guiding socket 120, and the guiding socket 120 is further rotated, and then the sharp protrusions 124 formed on the fastening portion 121 of the guiding socket 120 are firmly fastened to the upper end surface of the fastening screw 2.

As shown FIG. 4B, after the guiding socket 120 is fastened to the implant 1 such that the sharp protrusions 124 formed on the fastening portion 121 are firmly fastened to the upper end surface of the fastening screw 2, the working tool 3 is removed. After that, the drill bit 110 is mounted to the guiding socket 120 such that the hole drilling portion 111 and the cool water supplying portion 112 of the hole drilling drill bit 110 are inserted in the guiding socket 120. At this time, even not shown, the hand-piece connecting portion 114 of the drill bit 110 is mounted to a hand-piece to be operated.

The drill bit 110 is rotated by the operation of the hand-piece and it slowly drills a hole on the upper end surface of the broken fastening screw 2, and then it drills the enough depth of hole when the entrance restrain portion 113 reaches to the upper end surface of the guiding socket 120.

At this time, because the sharp protrusions 124 formed on the fastening portion 121 firmly holds to the upper end surface of the fastening screw 2, the broken fastening screw 2 is not rotated together with the drill bit 110 so that the hole can be easily drilled. After the hole is drilled with an enough depth, the drill bit 110 is removed and then the guiding socket 120 is also removed. Further, at this time, to cool the heat produced during drilling, the cooling water is supplied to the hole drilling portion 111 of the drill bit 110 and the surroundings thereof from outside through the cooling water supplying portion 112.

As shown in FIGs. 4A and 4B, when the guiding socket 120 is used to drill the fastening screw 2, the drilling central axis of the drill bit 110 will fit the central axis of the fastening screw 2, therefore the hole lies always on the central axis of the fastening screw 2.

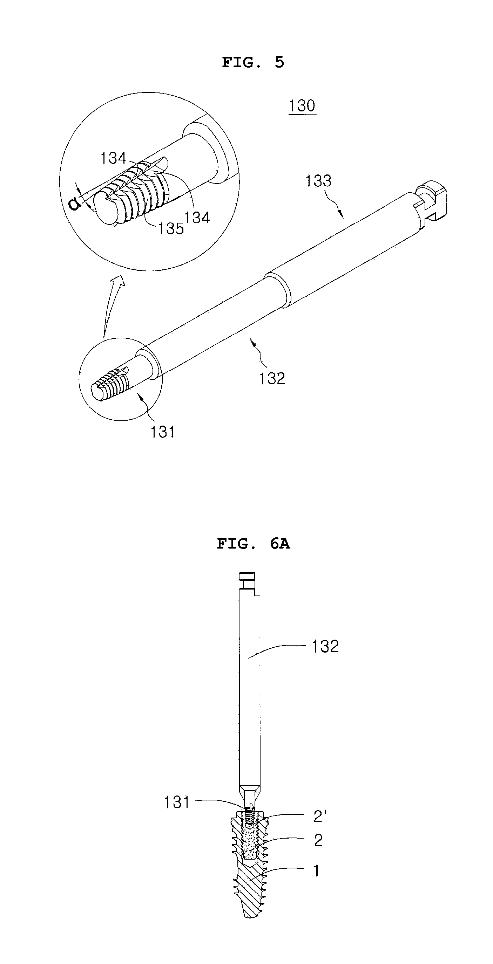

Next, referring to FIG. 5, it is explained a screw lifting drill bit for removing the fastening screw formed with the hole. FIG. 5 is a perspective view illustrating a screw lifting drill bit according to one preferred embodiment of the present invention.

As shown in FIG. 5, the screw lifting drill bit 130 includes a hole fastening portion 131, a connecting portion 132 and a hand-piece connecting portion 133.

The hole fastening portion 131 is for being fastened to the hole formed in the fastening screw, and it has a slight larger diameter than that of the hole drilling portion 111 of the drill bit 110 such that it can be well fastened to the hole. That is, the diameter of the hole fastening portion 131 is slightly larger than that of the hole.

The hole fastening portion 131 is formed with side cutting blades 134 which cut the inner periphery surface of the hole to form texture on the inner periphery surface and side fixing blades 135 which burrow the texture to be fixed, on the outer periphery surface thereof. The portion formed with the side cutting blades 134 and the side fixing blade 135 has a tapered shape to be narrower towards its end, and the tapered angle **α** is preferably below 15°. As like this, because the hole fastening portion 131 is formed to have a tapered shape, the side fixing blades 135 are much strongly fixed to the texture formed on the hole of the fastening screw when the hole fastening portion 131 is much deeply fastened in the hole formed in the fastening screw.

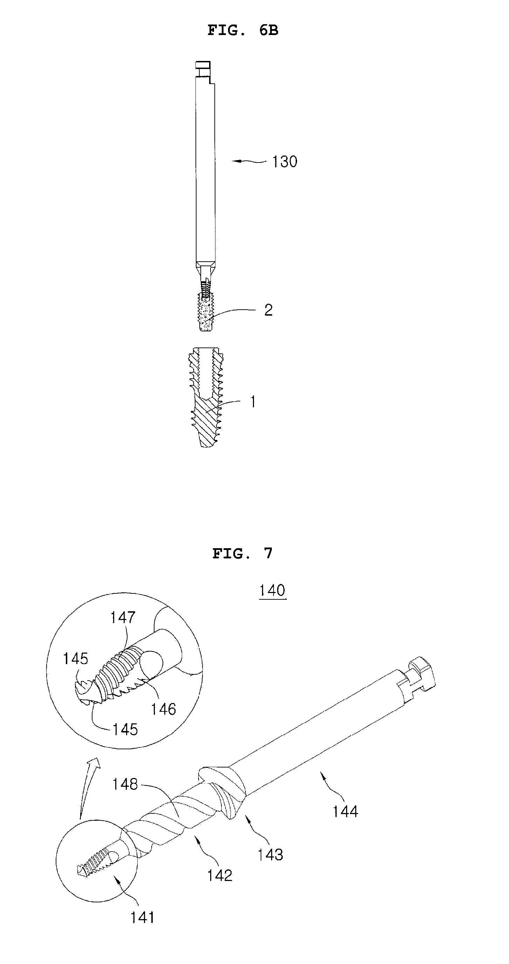

Next, referring to FIGs. 6A and 6B, it is explained a work to remove the broken or damaged fastening screw from the implant using the screw lifting drill bit in FIG. 5. FIGs. 6a and 6b are sectional views illustrating the work to remove the broken or damaged fastening screw from the implant using the screw lifting drill bit in FIG. 5.

As shown in FIG. 6A, the screw lifting drill bit 130 is positioned to fit the hole 2' formed on the center of the upper end portion of the fastening screw 2 which is remained in the implant 1, and the hole fastening portion 131 begins to be fastened to the hole 2' by rotating the screw lifting drill bit 130.

At this time, even not shown, the hand-piece connecting portion 133 of the drill bit 130 is firmly mounted to a hand-piece, and the direction of the drill bit 130 and the direction in which the hole fastening portion 131 is fastened to the hole 2' are opposite to the direction in which the fastening screw 2 is fastened to the implant 1.

In a condition like FIG. 6A, if the drill bit 130 is continually rotated, the hole fastening portion 131 is firmly fastened to the hole 2', and this fastening force is greater than the fastening force between the fastening screw 2 and the implant 1, therefore the fastening screw 2 is slowly released out from the implant 1 and then finally the fastening screw 2 is fully out from the implant 1 as shown in FIG. 6B.

As shown in FIG. 6B, the fastening screw 2 which is fully out from the implant 1 is still fastened to the hole fastening portion 131 of the drill bit 130. The fastening screw 2 is later removed from the drill bit 130 by the fingers of the user or a proper tool. As shown in FIG. 6B, after the fastening screw 2 is fully removed, the implant 1 is never damaged even little.

The drill bit for removing the implant fastening screw is composed of two drill bits, but alternatively various drill bits for removing the implant fastening screw composed of single drill bit are possible, and they will be explained below referring to FIGs. 7 to 14.

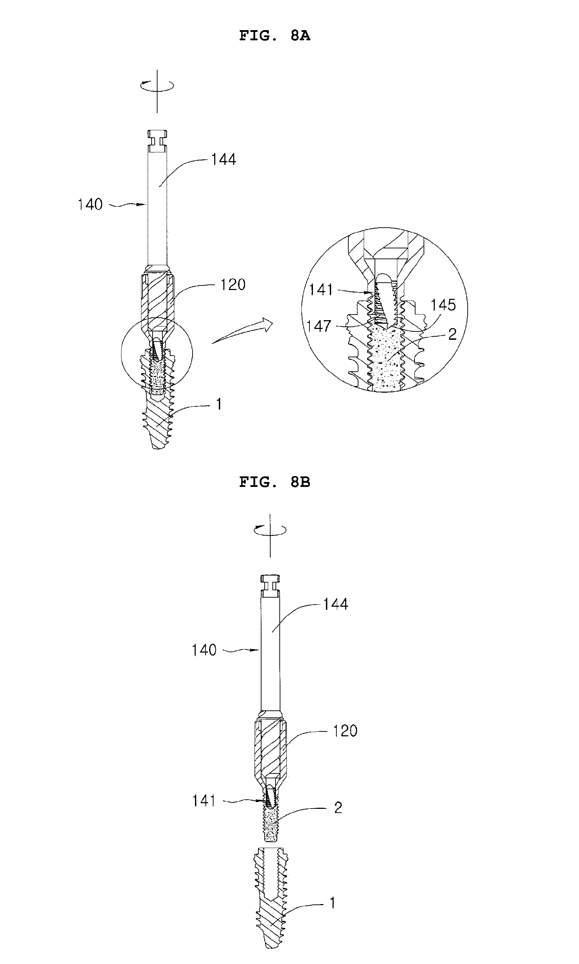

First, referring to FIGs. 7, 8A and 8B, it is explained a drill bit for removing an implant fastening screw according to another preferred embodiment of the present invention. FIG. 7 is a perspective view partially illustrating a drill bit for removing the implant fastening screw according to another preferred embodiment of the present invention.

As shown in FIG. 7, the drill bit 140 includes the screw removing portion 141, the cool water supplying portion 142, the entrance restrain portion 143 and the hand-piece connecting portion 144, which are integrally formed in order.

The screw removing portion 141 has a thin and short cylindrical shape. The screw removing portion 141 is formed with a pair of cutting blades 145 to be opposite with 180° on the end surface thereof and a pair of channels 146 to be connected to the pair of cutting blades 145 and to be twisted in longitudinal direction on the side surface thereof. The pair of cutting blades 145 axially drills the hole on the fastening screw, and the pair of channels 146 outwardly discharges the chips produced during drilling. At this time, the pair of cutting blades 145 is formed to do the cutting work in the direction that the fastening screw is released, and the pair of channels 146 is also formed to be twisted in the same direction.

Further, the screw removing portion 141 is formed with a pair of side fixing blades 147 on the outer periphery surface between the pair of channels 146. The pair of side fixing blades 147 burrows in the inner periphery surface of the hole drilled by the pair of cutting blades 145 to form texture and they are firmly fixed into the hole.

The cooling water supplying portion 142 is for supplying the cooling water to cool the heat produced during the drilling of the hole, and it has a thick and long cylindrical shape. The cooling water supplying portion 142 is spirally formed with the cooling water supplying groove 148 following to the outer periphery surface thereof to supply the cooling water from outside to the screw removing portion 141 and the surroundings of the cutting work. At this time, the spiral typed cooling water supplying groove 148 is formed in the direction in which the fastening screw is wound, that is, in the opposite direction of the direction in which the pair of cutting blades 145 or the pair of channels 146 is formed. To do like this, the cooling water can be much well supplied when the hole is drilled.

The entrance restraint portion 143 is for restraining the entrance depth of the screw removing portion 141 to drill the hole, and it is formed to have the larger diameter than the cooling water supplying portion 142 and to be stepped. In this embodiment, the entrance depth of the drill bit 140 is restrained by the entrance restraint portion 143 which is stepped from the cooling water supplying portion 142, but alternatively it may be marked to numerically show the entrance depth such that the user can easily know the entrance depth and then control the depth.

The hand-piece connecting portion 144 is for helping the drill bit 140 to be mounted to a working tool like a hand-piece.

Referring to FIGs. 8A and 8B, it is explained a work to remove the broken fastening screw remained the implant using the drill bit 140 in FIG. 7. FIGs. 8A and 8B are sectional views illustrating a work to remove the broken or damaged fastening screw remained the implant using the drill bit for removing implant fastening screw in FIG. 7 and the guiding socket in FIG. 2.

First, as shown in FIG. 8A, the guiding socket 120 is firmly fastened on the upper end portion of the implant 1 in which the broken fastening screw 2 is remained, and then the drill bit 140 is inserted into the guiding socket 120 until a pair of cutting blades 145 of the screw removing portion 141 reaches to the upper end surface of the fastening screw 2, and then the hole is begun to be drilled in the arrow direction (opposite to the fastening direction of the fastening screw) using the hand-piece. During drilling of the hole, the pair of side fixing blades 147 of the screw removing portion 141 burrows in the inner periphery surface of the hole to form texture and it begins to them to be firmly fixed into the hole. At this time, even not shown, the hand-piece connecting portion 144 of the drill bit 140 is mounted to a hand-piece to be operated, and the cooling water is supplied to the screw removing portion 141 of the drill bit 140 and the surroundings thereof from outside through the cooling water supplying portion 142.

The detailed explanation the work to fasten the guiding socket 120 will be omitted because it is identical with the explanation above mentioned in FIGs. 4A and 4B.

First, as shown in FIG. 8b, if the drill bit 140 is further rotated, the fastening force between the pair of side fixing blades 147 of the screw removing portion 141 and the fastening screw 2 becomes to be greater than the fastening force between the fastening screw 2 and the implant 1. Also, if the drill bit 140 is still further rotated, the fastening screw 2 is raised up together with the screw removing portion 141. Further, at this time, the guiding socket 120 firmly holding the upper end surface of the fastening screw 2 is also rotated and raised up together with the fastening screw 2.

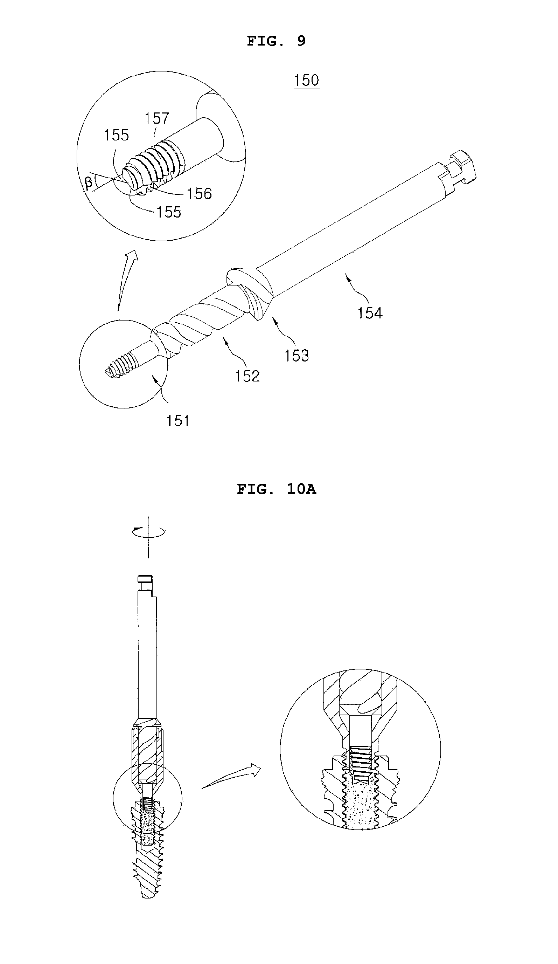

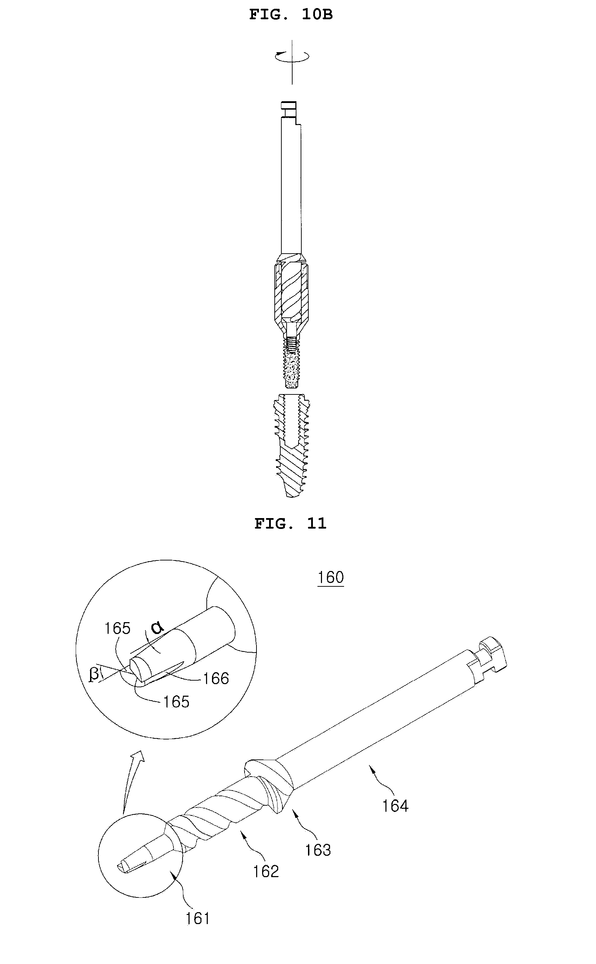

Next, referring to FIGs. 9, 10A and 10B, it is explained a drill bit for removing an implant fastening screw according to still another preferred embodiment of the present invention. FIG. 9 is a perspective view partially illustrating the drill bit for removing the implant fastening screw according to still another preferred embodiment of the present invention.

As shown in FIG. 9, the drill bit 150 includes the screw removing portion 151, the cooling water supplying portion 152, the entrance restrain portion 153 and the hand-piece connecting portion 154, which are integrally formed in order. The structure of the cooling water supplying portion 152, the entrance restrain portion 153 and the hand-piece connecting portion 154 is identical with that of the cooling water supplying portion 142, the entrance restrain portion 143 and the hand-piece connecting portion 144, and therefore they are not explained to avoid the repetition of the explanation.

The screw removing portion 151 is formed with a pair of cutting blades 155 to be contrary to each other on the end surface thereof and a pair of channels 156 to be straight in longitudinal direction on the side surface thereof. The pair of cutting blades 155 axially drills the hole on the fastening screw, and the pair of channels 156 outwardly discharges the chips produced during drilling. The pair of cutting blades 155 has a edge angle **β** below 90° to increase the cutting force.

Further, the screw removing portion 151 is formed with a pair of side fixing blades 157 on the outer periphery surface between the pair of channels 156. The pair of side fixing blades 157 burrows in the inner periphery surface of the hole drilled by the pair of cutting blades 155 to form texture and they are firmly fixed into the hole. The pair of side fixing blades 157 has the same shape as that of the pair of side fixing blades 147 in FIG. 7.

It is shown in FIGs. 10A and 10B a work to remove the broken fastening screw remained the implant using the drill bit 150 in FIG. 9. The work to remove the broken fastening screw is similar or nearly same as the work explained above referring to FIGs. 8A and 8B, it is not explained to avoid the repetition of the explanation.

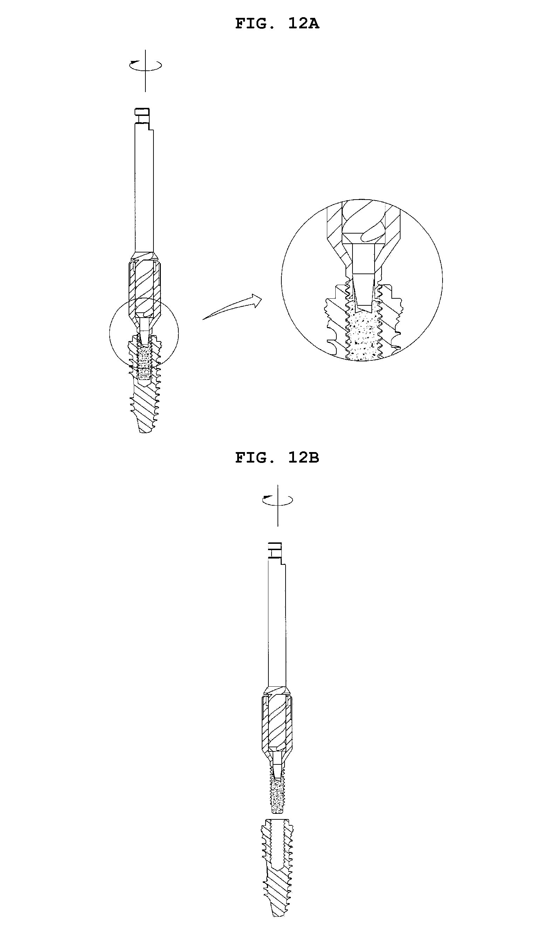

Next, referring to FIGs. 11, 12A and 12B, it is explained a drill bit for removing an implant fastening screw according to still another preferred embodiment of the present invention. FIG. 11 is a perspective view partially illustrating the drill bit for removing the implant fastening screw according to still another preferred embodiment of the present invention.

As shown in FIG. 11, the drill bit 160 includes the screw removing portion 161, the cooling water supplying portion 162, the entrance restrain portion 163 and the hand-piece connecting portion 164, which are integrally formed in order. The structure of the cooling water supplying portion 162, the entrance restrain portion 163 and the hand-piece connecting portion 164 is identical with that of the cooling water supplying portion 142, the entrance restrain portion 143 and the hand-piece connecting portion 144, and therefore they are not explained to avoid the repetition of the explanation.

The screw removing portion 161 is formed with a pair of cutting blades 165 to be contrary to each other on the end surface thereof and a pair of channels 166 to be straight in longitudinal direction on the side surface thereof. The pair of cutting blades 165 axially drills the hole on the fastening screw, and the pair of channels 166 outwardly discharges the chips produced during drilling. The pair of cutting blades 165 has a edge angle **β** below 90° to increase the cutting force. The tapered angle **α** of the screw removing portion 161 is preferably below 15°.

It is shown in FIGs. 12A and 12B a work to remove the broken fastening screw remained the implant using the drill bit 160 in FIG. 11. The work to remove the broken fastening screw is similar or nearly same as the work explained above referring to FIGs. 8A and 8B, and therefore it is not explained to avoid the repetition of the explanation.

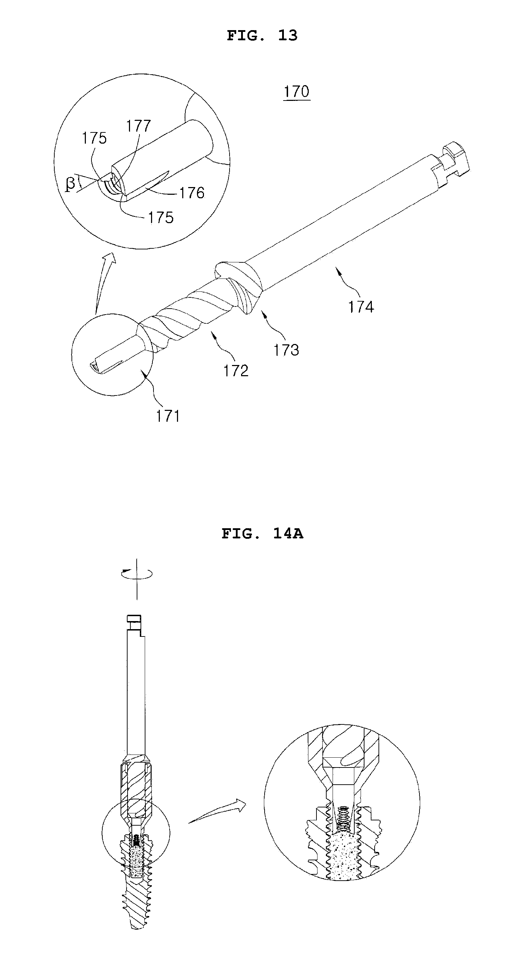

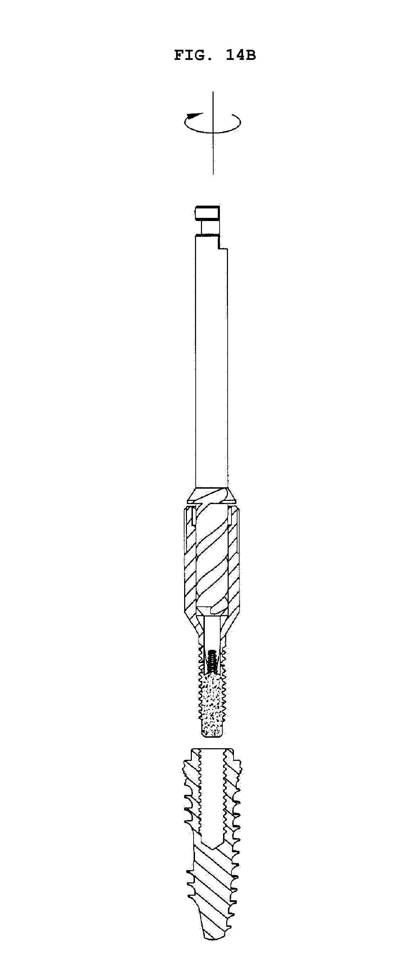

Finally, referring to FIGs. 13, 14A and 14B, it is explained a drill bit for removing an implant fastening screw according to still another preferred embodiment of the present invention. FIG. 13 is a perspective view partially illustrating the drill bit for removing the implant fastening screw according to still another preferred embodiment of the present invention.

As shown in FIG. 13, the drill bit 170 includes the screw removing portion 171, the cooling water supplying portion 172, the entrance restrain portion 173 and the hand-piece connecting portion 174, which are integrally formed in order. The structure of the cooling water supplying portion 172, the entrance restrain portion 173 and the hand-piece connecting portion 174 is identical with that of the cooling water supplying portion 142, the entrance restrain portion 143 and the hand-piece connecting portion 144, and therefore they are not explained to avoid the repetition of the explanation.

The screw removing portion 171 is hollow and it is formed with a pair of cutting blades 175 to be contrary to each other on the end surface thereof and a pair of channels 176 to be straight in longitudinal direction on the side surface thereof. The pair of cutting blades 175 axially drills the annular hole on the fastening screw, and the pair of channels 176 outwardly discharges the chips produced during drilling. The pair of cutting blades 175 has a edge angle **β** below 90° to increase the cutting force. Further, the screw removing portion 171 is formed with a pair of side fixing blades 177 on the inner periphery surface between the pair of channels 176.

The screw removing portion 171 drills the hole in annular shape, therefore the hole is formed with a cylindrical protrusion. The cylindrical protrusion is fixed with the side fixing blades 177 to be covered.

It is shown in FIGs. 14A and 14B a work to remove the broken fastening screw remained the implant using the drill bit 170 in FIG. 13. The work to remove the broken fastening screw is similar or nearly same as the work explained above referring to FIGs. 8A and 8B, and therefore it is not explained to avoid the repetition of the explanation.

Although a few embodiments of the present invention have been shown and described, the present invention is not limited to the described embodiments. Instead, it would be appreciated by those skilled in the art that changes may be made to these embodiments without departing from the principles and spirit of the invention, the scope of which is defined by the claims and their equivalents.

## Claims

1. A drill bit for removing implant fastening screw comprising:
a hole drilling drill bit for axially drilling a hole in the fastening screw which is broken or damaged in an implant; and
a screw lifting drill bit for being firmly fixed the hole of the fastening screw and for lifting the fastening screw.

2. The drill bit according to claim 1, wherein the hole drilling drill bit is provided with a hole drilling portion to drill the hole and a cooling water supplying portion to supply the cooling water to the hole drilling portion to cool the heat produced during the drilling of the hole, which are integrally provided in order.

3. The drill bit according to claim 2, wherein an entrance restrain portion to restrain the entrance depth of the hole drilling portion to drill the hole is provide to be integral very after the cooling water supplying portion.

4. The drill bit according to claim 3, wherein a hand-piece connecting portion to be mounted to a working tool like a hand-piece is further provide to be integral very after the entrance restrain portion.

5. The drill bit according to one of claims 2 to 4, wherein the hole drilling portion is formed with a pair of cutting blades for drilling the hole on the fastening screw, the cutting blades being opposite with each other on the end surface thereof.

6. The drill bit according to claim 5, wherein the hole drilling portion is formed with a pair of channels for discharging the drilled chips of the fastening screw, the channels being connected to the pair of cutting blades and twisted in longitudinal direction on the side surface thereof.

7. The drill bit according to one of claims 2 to 4, wherein the cooling water supplying portion is spirally formed with a cooling water supplying groove, and the spiral cooling water supplying groove is formed to have the opposite direction of the direction in which the pair of cutting blades or the pair of channels is formed such that the cooling water is well supplied.

8. The drill bit according to claim 1, wherein the screw lifting drill bit is provided with a hole fastening portion to be fastened to the hole formed in the fastening screw, and the diameter of the hole fastening portion is greater than that of the hole.

9. The drill bit according to claim 8, wherein the hole fastening portion is formed with side cutting blades which cut the inner periphery surface of the hole to form texture on the inner periphery surface and side fixing blades which burrow the texture to be fixed, on the outer periphery surface thereof.

10. The drill bit according to claim 9, wherein the portion formed with the side cutting blades and the side fixing blades has a tapered shape to be narrower towards its end.

11. The drill bit according to claim 10, wherein the tapered angle is preferably below 15°.

12. A drill bit for removing an implant fastening screw including a screw removing portion to remove the fastening screw broken or damaged in an implant, the screw removing portion is composed of:
a pair of cutting blades for axially drilling a hole on the fastening screw, the cutting blades being opposite with each other on the end surface thereof;
a pair of channels for outwardly discharging the chips produced during drilling on the side surface thereof, the channels being connected to the pair of cutting blades and being twisted in longitudinal direction on the side surface thereof; and
a pair of side fixing blades for burrowing in the inner periphery surface of the hole drilled by the pair of cutting blades to form texture and being firmly fixed into the hole, the side fixing blades formed to be between the pair of channels.

13. The drill bit according to claim 12, wherein the pair of cutting blades does the cutting work in the direction that the fastening is released, and the pair of channels is twisted in the direction that the fastening is released.

14. A drill bit for removing implant fastening screw including a screw removing portion to remove the fastening screw broken or damaged in an implant, the screw removing portion is composed of:
a pair of cutting blades for axially drilling a hole on the fastening screw, the cutting blades being contrary to each other on the end surface thereof;
a pair of channels for outwardly discharging the chips produced during drilling on the side surface thereof, the channels being connected to the pair of cutting blades and being straight in longitudinal direction on the side surface thereof;
and
a pair of side fixing blades for burrowing in the inner periphery surface of the hole drilled by the pair of cutting blades to form texture and being firmly fixed in the hole, the side fixing blades formed to be between the pair of channels.

15. The drill bit according to claim 14, wherein the pair of cutting blades does the cutting work in the direction that the fastening is released.

16. The drill bit according to claim 14, wherein the pair of cutting blades has a edge angle of below 90° to increase the cutting force.

17. A drill bit for removing an implant fastening screw including a screw removing portion to remove the fastening screw broken or damaged in an implant, the screw removing portion is composed of:
a pair of cutting blades for axially drilling a hole on the fastening screw, the cutting blades being contrary to each other on the end surface thereof; and
a pair of channels for outwardly discharging the chips produced during drilling on the side surface thereof, the channels being connected to the pair of cutting blades and being straight in longitudinal direction on the side surface thereof, wherein
the screw removing portion is formed to be tapered to have an angle of below 15°.

18. The drill bit according to claim 17, wherein the pair of cutting blades has a edge angle of below 90° to increase the cutting force.

19. A drill bit for removing an implant fastening screw including a screw removing portion to remove the fastening screw broken or damaged in an implant, the screw removing portion is hollow and composed of:
a pair of cutting blades for axially drilling one annular hole on the fastening screw, the cutting blades being contrary to each other on the end surface thereof;
a pair of channels for outwardly discharging the chips produced during drilling on the side surface thereof, the channels being connected to the pair of cutting blades and being straight in longitudinal direction on the side surface thereof; and
a pair of side fixing blades for burrowing in the inner periphery surface of the annular hole drilled by the pair of cutting blades to form texture and being firmly fixed into the hole, the side fixing blades formed to be between the pair of channels.

20. The drill bit according to claim 19, wherein the pair of cutting blades has a edge angle of below 90° to increase the cutting force.

21. A guiding socket comprising:
a fastening portion for being firmly fastened in the upper end portion of an implant in which a broken or damaged fastening screw is remained; and
a guiding portion for guiding a hole drilling drill bit to fit the central axis of the fastening screw and the central axis of the hole drilling drill bit.

22. The drill bit according to claim 21, wherein the fastening portion has a hollow cylindrical shape, and it is formed with threads on the outer periphery surface to be corresponded to the threads formed in the inner periphery surface of the implant.

23. The drill bit according to claim 22, wherein the threads has the length of 0.2mm∼5mm.

24. The drill bit according to claim 22, wherein the fastening portion is formed with sharp protrusions for firmly hold the upper end surface of the fastening screw, the fastening portion being on the end surface following to the circumference thereof.

25. The drill bit according to claim 21, wherein the guiding portion is formed with a hexagonal shaped installing recess to be inserted with a hexagonal shaped installing protrusion formed on the working tool, the guiding portion being on the inner periphery surface of the end portion thereof.
